# EUROPEAN PATENT APPLICATION

(11) **EP 1 721 902 A1**
(43) Date of publication of application: **15.11.2006**
(21) Application number: 06252437.6
(22) Date of filing: 08.05.2006
(51) Int. Cl.: C07D 277/12, C07D 277/18, A61K 31/426

(54) **Production methods of thiazoline compounds and optically active alpha-alkylcysteine**

(30) Priority: 09.05.2005 JP 2005136695
(71) Applicant: Ajinomoto Co., Inc., Tokyo 104-8315 (JP)
(72) Inventor: Hamada, Takayuki, Kawasaki-shi, Kanagawa 210-8681 (JP); Yatagai, Masanobu, Kawasaki-shi, Kanagawa 210-8681 (JP)
(74) Representative: Nicholls, Kathryn Margaret

(57) **Abstract**

An optically active compound (I) is reacted with compound (II) to give optically active compound (III), which is subjected to alkali hydrolysis and deprotection when R¹ is alkyl: 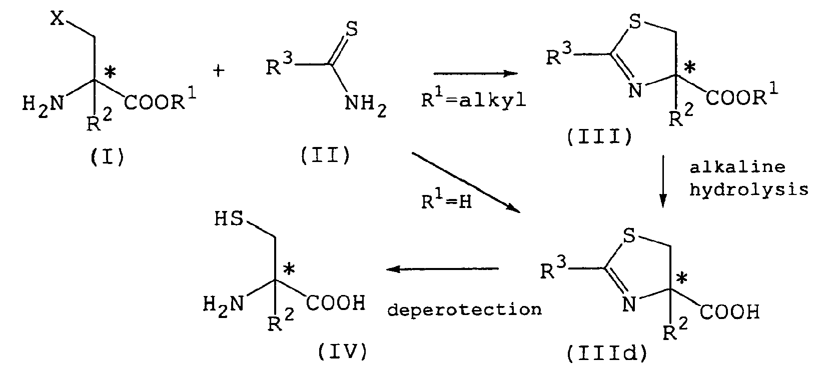 wherein X is a chlorine atom and the like, and R¹, R² and R³ are each an alkyl group and the like, or
racemic compound (I) is reacted with compound (II) to give racemic compound (III), which is asymmetrically hydrolyzed by an enzyme to perform optical resolution, and subjected to alkali hydrolysis and deprotection: 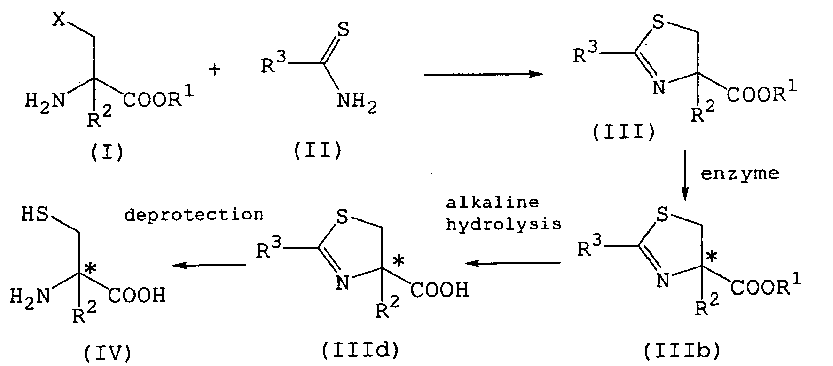 wherein X is a chlorine atom and the like and R¹, R² and R³ are each an alkyl group and the like.

## Description

### TECHNICAL FIELD OF THE INVENTION

The present invention relates to a production method of an optically active α-alkylcysteine useful as an intermediate for pharmaceutical products. Moreover, the present invention relates to a novel production method of a thiazoline compound useful as an intermediate for producing the optically active α-alkylcysteine.

### BACKGROUND OF THE INVENTION

An optically active α-alkylcysteine is useful as a synthetic intermediate for pharmaceutical products. For example, α-methyl-L-cysteine is useful as a synthetic intermediate for anti-inflammatory agents having a nitric oxide synthase inhibitory activity (WO 2004/039772).

As shown in the following scheme, a method comprising subjecting a thiazoline compound, which is obtained by Michael addition reaction of sulfur atom of thiourea to β-unsaturated carboxylic acid compound, to an enzyme reaction has been used for the synthesis of L-cysteine. However, when the α-position is an alkyl group, β-unsaturated carboxylic acid cannot be formed as an intermediate, and therefore, this method cannot be applied to α-alkylcysteine.

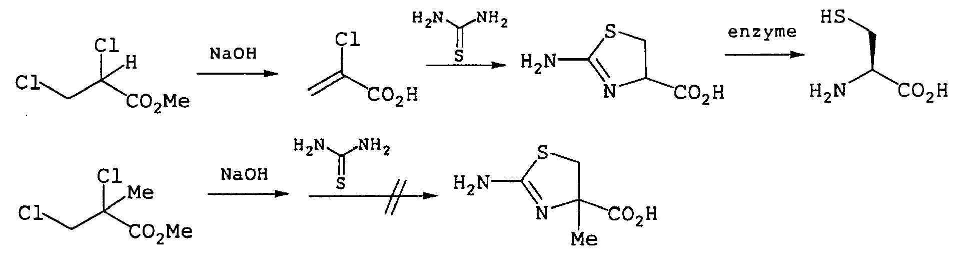

The production method of an optically active α-alkylcysteine is, therefore, mostly a nucleophilic substitution reaction with thiol and thioacetic acid or a method using cysteine as a starting material.

For example, a method using an alanine ester as a starting material, as shown in the following scheme, has been reported (Synthesis, 1983, p. 37).

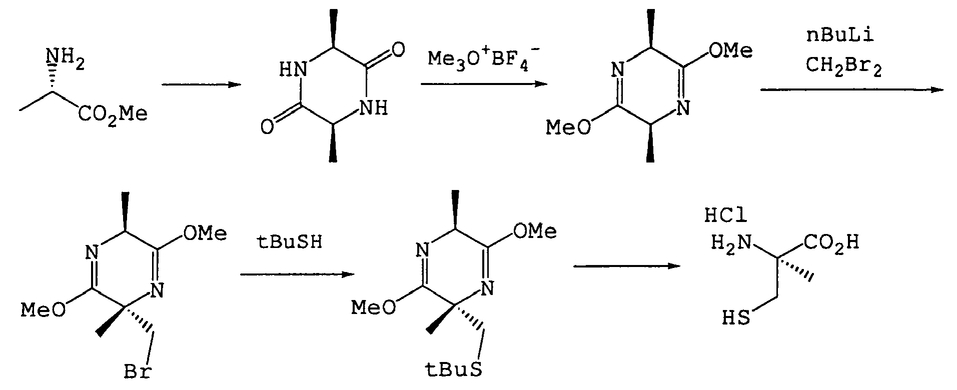

In this method, an alanine ester is dimerized for the protection of an amino group. However, the efficiency is low, since one molecule thereof is not converted to α-methylcysteine.

Moreover, since the method requires use of an expensive reagent such as n-butyllithium and dibromomethane or a reagent with a stinky odor such as tert-butylmercaptane, it is not suitable for industrial production.

As a different method, a method shown in the following scheme, which uses cysteine ester as a starting material to go through a thiazoline compound, has been reported (JP-A-2003-201284) .

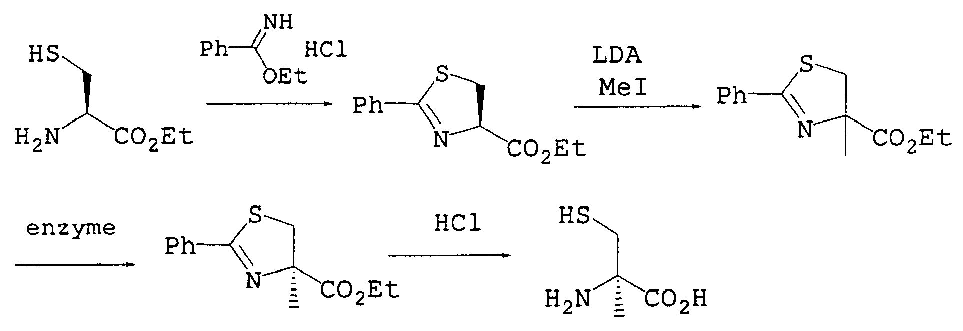

According to this method, lithium diisopropylamide, which is expensive and requires a reaction at a low temperature, is used for the methylation step of a thiazoline ring. Moreover, since a chiral source of cysteine is lost during methylation, optical resolution by an enzyme reaction or chiral amine is necessary.

### SUMMARY OF THE INVENTION

It is therefore an object of the present invention to provide an industrially efficient production method of an optically active α-alkylcysteine useful as a synthetic intermediate for pharmaceutical products.

In an attempt to achieve the aforementioned object, the present inventors have conducted intensive studies of a production method of a thiazoline compound which is an effective intermediate for the production of α-alkylcysteine.

As a result, they have surprisingly found that the desired thiazoline derivative can be obtained conveniently and in a high yield by reacting an α-alkyl-β-haloalanine derivative and thioamides or thioureas, which resulted in the completion of the present invention.

Accordingly, the present invention provides the following.
[1] A production method of a compound represented by the formula (III): 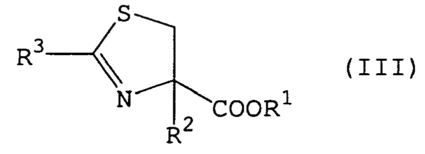 wherein R¹ is a hydrogen atom or an alkyl group, R² is an alkyl group, and R³ is an alkyl group, an allyl group, an aryl group or an amino group (hereinafter to be also referred to as compound (III)) or a salt thereof, which comprises reacting a compound represented by the formula (I): 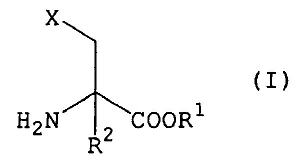 wherein R¹ and R² are as defined above, and X is a chlorine atom, a bromine atom or an iodine atom (hereinafter to be also referred to as compound (I)) or a salt thereof with a compound represented by the formula (II): 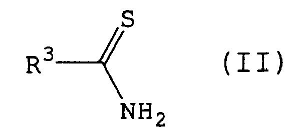 wherein R³ is as defined above (hereinafter to be also referred to as compound (II)).
[2] The production method of the above-mentioned [1], wherein compound (I) is a racemate.
[3] The production method of the above-mentioned [2], wherein R¹ is an alkyl group.
[4] A production method of a compound represented by the formula (IIIb) : 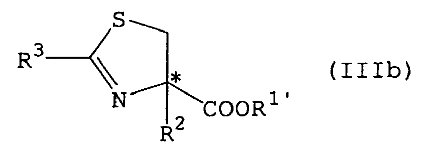 wherein R¹' and R² are the same or different and each is an alkyl group, R³ is an alkyl group, an allyl group, an aryl group or an amino group, and * shows a chiral carbon atom (hereinafter to be also referred to as compound (IIIb)) or a salt thereof and a compound represented by the formula (IIIc): 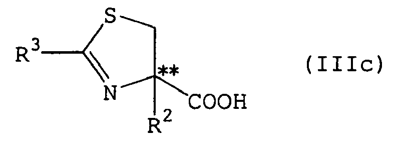 wherein ** shows a chiral carbon atom of an S configuration when * shows an R configuration, and a chiral carbon atom of an R configuration when * shows an S configuration, and other symbols are as defined above (hereinafter to be also referred to as compound (IIIc)) or a salt thereof, which comprises reacting a compound represented by the formula (IIIa): 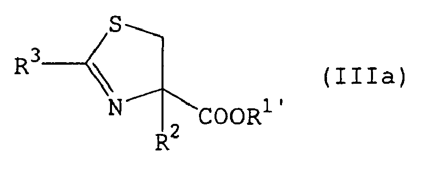 wherein each symbol is as defined above (hereinafter to be also referred to as compound (IIIa)), which is obtained by the method described in the above-mentioned [3], or a salt thereof with an enzyme having an ability to asymmetrically hydrolyze the compound.
[5] A production method of a compound represented by the formula (IIId) : 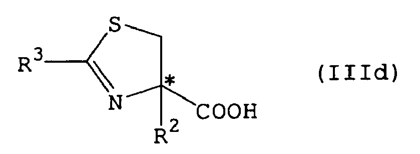 wherein each symbol is as defined above (hereinafter to be also referred to as compound (IIId)) or a salt thereof, which comprises alkali hydrolysis of an ester moiety of compound (IIIb) obtained by the method described in the above-mentioned [4] or a salt thereof.
[6] A production method of a compound represented by the formula (IVa) : 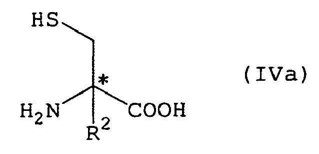 wherein R² is as defined above (hereinafter to be also referred to as compound (IVa)) or a salt thereof, which comprises reacting compound (IIId) obtained by the method described in the above-mentioned [5] or a salt thereof with an acid for deprotection.
[7] A production method of a compound represented by the formula (IVb) : 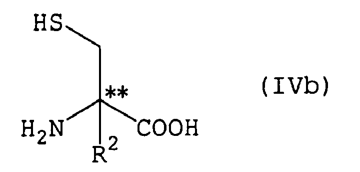 wherein R² is as defined above (hereinafter to be also referred to as compound (IVb)) or a salt thereof, which comprises reacting compound (IIIc) obtained by the method described in the above-mentioned [4] or a salt thereof with an acid for deprotection.
[8] The production method of any one of the above-mentioned [1]-[7], wherein R³ is a methyl group.
[9] The production method of any one of the above-mentioned [4]-[8], wherein R¹' is a methyl group or an ethyl group.
[10] The production method of any one of the above-mentioned [1]-[9], wherein R² is a methyl group.
[11] The production method of the above-mentioned [1], wherein compound (I) is an optically active form.
[12] The production method of the above-mentioned [11], wherein R¹ is a hydrogen atom.
[13] The production method of the above-mentioned [11], wherein R¹ is an alkyl group.
[14] A production method of a compound represented by the formula (IIIf): 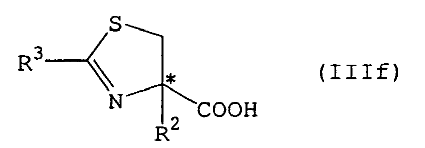 wherein R² is an alkyl group, R³ is an alkyl group, an allyl group, an aryl group or an amino group, and * shows a chiral carbon atom (hereinafter to be also referred to as compound (IIIf)) or a salt thereof, which comprises alkali hydrolysis of an ester moiety of a compound represented by the formula (IIIe): 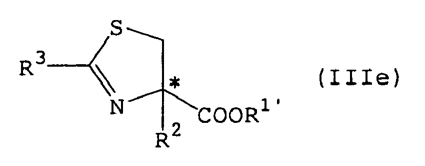 wherein R^{1'} is an alkyl group, and other symbols are as defined above (hereinafter to be also referred to as compound (IIIe)), which is obtained by the method described in the above-mentioned [13], or a salt thereof.
[15] A production method of a compound represented by the formula (IV): 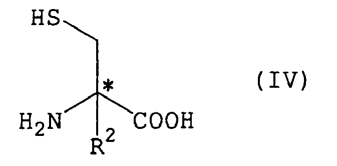 wherein R² is as defined above (hereinafter to be also referred to as compound (IV)) or a salt thereof, which comprises reacting compound (IIIf) obtained by the method described in the above-mentioned [12] or [14] or a salt thereof with an acid for deprotection.
[16] The production method of any one of the above-mentioned [11]-[15], wherein R³ is a methyl group.
[17] A production method of a compound represented by the formula (IIIh): 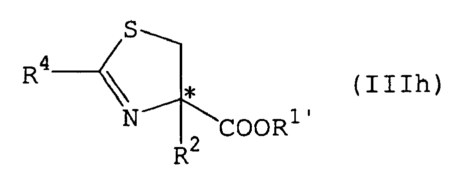 wherein R^{1'}, R² and R⁴ are the same or different and each is an alkyl group, and * shows a chiral carbon atom (hereinafter to be also referred to as compound (IIIh)) or a salt thereof and a compound represented by the formula (IIIi): 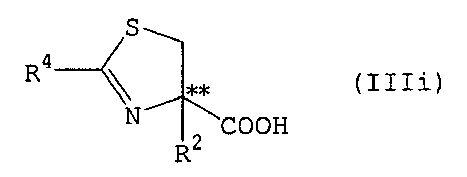 wherein ** shows a chiral carbon atom of an S configuration when * shows an R configuration, and a chiral carbon atom of an R configuration when * shows an S configuration, and other symbols are as defined above (hereinafter to be also referred to as compound (IIIi)) or a salt thereof, which comprises reacting a compound represented by the formula (IIIg): 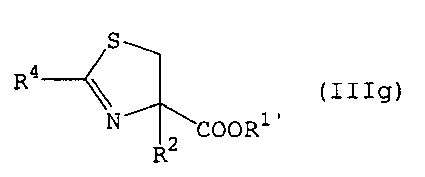 wherein each symbol is as defined above (hereinafter to be also referred to as compound (IIIg)) with an enzyme having an ability to asymmetrically hydrolyze the compound.
[18] A production method of a compound represented by the formula (IIIj): 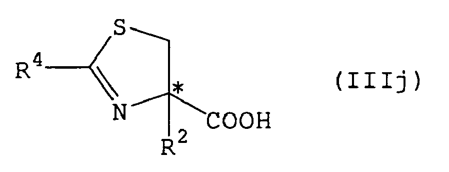 wherein each symbol is as defined above (hereinafter to be also referred to as compound (IIIj)) or a salt thereof, which comprises alkali hydrolysis of an ester moiety of compound (IIIh) obtained by the method described in the above-mentioned [17] or a salt thereof.
[19] A production method of compound (IVa) or a salt thereof, which comprises reacting compound (IIIj) obtained by the method described in the above-mentioned [18] or a salt thereof with an acid for deprotection.
[20] A production method of compound (IVb) or a salt thereof, which comprises reacting compound (IIIi) obtained by the method described in the above-mentioned [17] or a salt thereof with an acid for deprotection.
[21] The production method of any one of the above-mentioned [17]-[20], wherein R³ is a methyl group.
[22] The production method of any one of the above-mentioned [17]-[21], wherein R¹' is a methyl group or an ethyl group.
[23] The production method of any one of the above-mentioned [17]-[22], wherein R² is a methyl group.
[24] A compound represented by the formula (IIIk): 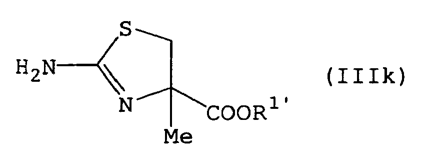 wherein R¹' is an alkyl group (hereinafter to be also referred to as compound (IIIk)) or a salt thereof.
[25] A compound represented by the formula (IIIl): 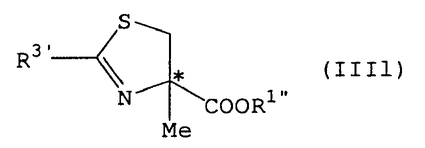 wherein R¹" is a hydrogen atom or a methyl group, R³_{,} is an alkyl group, an allyl group or an amino group, and * shows a chiral carbon atom (hereinafter to be also referred to as compound (IIIl)) or a salt thereof.
[26] A compound represented by the formula (IIIm): 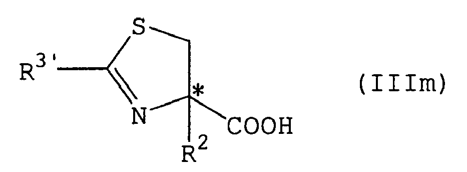 wherein R² is an alkyl group, R³' is an alkyl group, an allyl group or an amino group, and * shows a chiral carbon atom (hereinafter to be also referred to as compound (IIIm)) or a salt thereof.

According to the present invention, a high yield and convenient production method of a thiazoline compound useful as an intermediate for α-alkylcysteine, namely, compound (III), is provided (the above-mentioned [1] and the like).

When the thus-obtained compound (III) is a racemate, it can be optically resolved with ease by asymmetric hydrolysis by an enzyme (the above-mentioned [4] and the like), and when the optically active compound (I) is used as a starting material, optically active compound (III) can be obtained without optical resolution (the above-mentioned [11] and the like).

Moreover, optically active α-alkylcysteine, namely, compound (IV), can be obtained by alkali hydrolysis of the optically active compound (III) to give a carboxylic acid derivative, and deprotection thereof with an acid (the above-mentioned [5], [6], [14], [15] and the like).

The production method of the thiazoline compound and optically active α-alkylcysteine of the present invention does not use n-butyllithium, lithium diisopropylamide and the like, which are expensive and require cryogenic reaction, and is not requested to use mercaptanes having a stinky odor. Therefore, the method is suitable for industrial production as compared to conventional methods.

Of the asymmetric hydrolyses of compound (III) by an enzyme, an embodiment where the 2-position of thiazoline ring is an alkyl group is novel (the above-mentioned [17]). An embodiment where the 2-position of thiazoline ring is an alkyl group (particularly, methyl group) is superior in the following points as compared to an embodiment of a phenyl group, and is an industrially preferable method.
(1) The cost is low and the embodiment is economically advantageous.
(2) In the enzyme reaction, the substrate shows high water-solubility, and co-use of an organic solvent is not necessary.
(3) Deprotection of thiazolidine ring with an acid at a later stage proceeds under milder conditions.
(4) Carboxylic acids (acetic acid and the like) generated as a by-product in the deprotection with an acid can be easily removed from the object product.

### DETAILED DESCRIPTION OF THE INVENTION

The present invention is explained in detail in the following.

As the "alkyl group" for R¹, R¹', R², R³, R³' or R⁴, a straight chain or branched alkyl group having 1 to 10 carbon atoms, preferably 1 to 4 carbon atoms, such as methyl group, ethyl group, propyl group, isopropyl group, butyl group, isobutyl group, sec-butyl group, tert-butyl group, pentyl group, isopentyl group, neopentyl group, hexyl group, heptyl group, octyl group, nonyl group, decyl group and the like can be mentioned.

As the "aryl group" for R³ or R³', an aryl group having 6 to 10 carbon atoms, such as phenyl group, 1- or 2-naphthyl group and the like can be mentioned.

The carbon atoms marked with * or ** are chiral carbon atoms having any configuration. In asymmetric hydrolysis of compound (III) by an enzyme, however, since one having either configuration of racemic substrate is selectively or preferentially hydrolyzed, when * shows an S configuration, ** shows the opposite R configuration, and when * shows an R configuration, ** shows an S configuration.

Compounds (I), (IIIk), (IV), (IVa) and (IVb) have an amino group, and may form inorganic acid salts (e.g., hydrochloride, sulfate and the like), organic acid salts (e.g., acetate, trifluoroacetate, tosylate, mesylate and the like) and the like.

When compounds (III), (IIIa), (IIIb), (IIIc), (IIId), (IIIe), (IIIf), (III1) and (IIIm) have an amino group, similar salts may be formed.

Compounds (IIIc), (IIId), (IIIf), (IIIi), (IIIj), (IIIm), (IV), (IVa) and (IVb) have a carboxyl group, and they may form alkali metal salts (e.g., potassium salt, sodium salt, lithium salt and the like), organic amine salts (e.g., triethylamine salt, dicyclohexylamine salt and the like) and the like.

When compound (IIIl) has a carboxyl group, similar salts may be formed.

The salts of the compound of the present invention encompass hydrates, solvates (e.g., methanol solvate, ethanol solvate, ether solvate) and the like.

R¹ and R^{1'} are preferably a methyl group or an ethyl group, in consideration of the selectivity of the enzyme reaction and water-solubility of the substrate.

Since α-methyl-L-cysteine is a useful synthetic intermediate for anti-inflammatory agents, an embodiment wherein R² is a methyl group is preferable.

As R³ and R⁴, a methyl group is preferable for the following reasons (1)-(4).
(1) The cost is low and the embodiment is economically advantageous.
(2) In the enzyme reaction, the substrate shows high water-solubility, and co-use of an organic solvent is not necessary.
(3) Deprotection of thiazolidine ring with an acid at a later stage proceeds under milder conditions.
(4) Acetic acid generated as a by-product in the deprotection with an acid can be easily removed from the object product.

The outline of the present invention is shown in the following scheme. 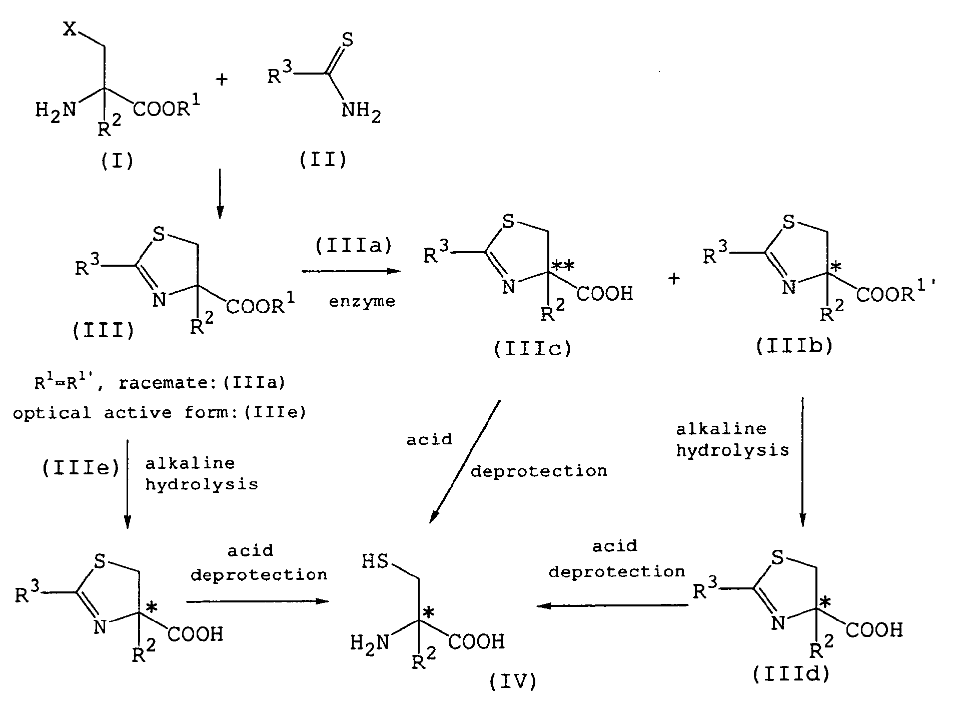 wherein each symbol is as defined above.

A novel and the most characteristic step in the present invention is a step for obtaining thiazoline compound (III) by reacting β-halo-α-alkylaniline compound (I) with thioamide or thiourea compound (II) (hereinafter sometimes to be also referred to as thiazoline cyclization step in this specification).

The thiazoline cyclization step is industrially advantageous as compared to the conventional methods, since (1) thiourea and thioamides (e.g., thioacetamide), which are starting materials, are solids, easy to handle, give less sulfur odor and are inexpensive; and (2) a Cl group having low reactivity as a leaving group can be converted to an S group.

Conventionally, the reaction of this kind has been carried out by production of β-unsaturated carboxylic acid resulting from β elimination of chlorine atom and subsequent Michael addition of sulfur atom. However, since compound (I) does not have a hydrogen atom at the α-position, β-unsaturated carboxylic acid is not involved.

Since the thiazoline cyclization step is free from β elimination, when an optically active form is used as compound (I), an optically active compound (III) can be obtained while maintaining its configuration.

When the thus-obtained compound (III) is a racemate, optically active α-alkylcysteine, i.e., compound (IV), can be obtained by, after optical resolution by asymmetric hydrolysis by an enzyme (hereinafter sometimes to be also referred to as an asymmetric hydrolysis step in this specification), (1) hydrolyzing optically active compound (IIIb), which is recovered without asymmetric hydrolysis, with an alkali to give compound (IIId) (hereinafter sometimes to be also referred to as an alkali hydrolysis step in this specification), which is then deprotected with an acid (hereinafter sometimes to be also referred to as a deprotection step in this specification), or (2) directly subjecting optically active compound (IIIc) hydrolyzed asymmetrically to a deprotection step.

When optically active compound (III) is obtained using optically active compound (I) as a starting material in the thiazoline cyclization step, it can be directly subjected to an alkali hydrolysis step and a deprotection step with an acid, without optical resolution to give compound (IV). Each step is explained in detail in the following.

### 1. thiazoline cyclization step

Compound (III) can be obtained, for example, by mixing compound (I) and compound (II) in a solvent. The order of addition of the reagents is not particularly limited.

Compound (I) to be used for the thiazoline cyclization step can be synthesized by a known method. For example, racemic compound (I) can be prepared by the method described in J. Org. Chem., Vol. 44, No. 15, p. 2732-2742 (1979) or JP-A-6-199755.

An optically active compound (I) can be prepared by the method described in Journal of Natural Sciences and Mathematics, Vol. 30, No. 2, p. 83-91 (1990). Using the optically active compound (I), optically active compound (III) retaining the configuration thereof can be obtained.

As compound (II), a commercially available product can be used.

The amount of compound (II) to be used is generally 0.5 mol - 1.5 mol, preferably 0.8 mol - 1.0 mol, per 1 mol of compound (I).

The solvent is not particularly limited as long as it does not inhibit the reaction and, for example, alcohols (e.g., methanol, ethanol, 1-propanol, 2-propanol etc.), water, N,N-dimethylformamide (DMF), N,N-dimethylacetamide (DMA), dimethyl sulfoxide (DMSO), N-methylpyrrolidone (NMP) and the like and a mixed solvent thereof can be mentioned, with preference given to methanol, water and the like.

The amount of the solvent to be used is generally 1-fold weight to 10-fold weight, preferably 2-fold weight to 5-fold weight, of the total amount of compound (I) and compound (II) to be used.

The thiazoline cyclization step is generally performed at a temperature within the range of from 40°C to the reflux temperature of the solvent to be used (preferably 50°C-100°C). The reaction time is generally 1 hr-24 hr (preferably 2 hr-16 hr) within the above-mentioned temperature range.

Compound (III) can be isolated and purified by a conventional method. For example, compound (III) can be isolated by evaporating the solvent after the completion of the reaction as necessary, extracting the residue with an organic solvent, washing the organic layer successively with water, aqueous acidic solution (e.g., hydrochloric acid, sulfuric acid and the like), aqueous alkali solution (e.g., saturated aqueous sodium hydrogen carbonate, brine and the like) and the like, and concentrating the partitioned organic layer.

Moreover, compound (III) can be purified by applying recrystallization or silica gel column chromatography. The compound may be subjected to the next step without purification.

Of the racemic compound (III) obtained in the thiazoline cyclization step, an embodiment wherein R¹ is an alkyl group, R² is a methyl group and R³ is an amino group, namely, compound (IIIk), is a novel compound and useful as a synthetic intermediate for pharmaceutical products and the like.

### 2. Asymmetric hydrolysis step

The racemic compound (III) can be optically resolved by the action of an enzyme having an ability to asymmetrically hydrolyze the compound.

Here, the "asymmetric hydrolysis of compound (III)" means selective or preferential hydrolysis of the ester of one of the enantiomers of compound (III).

As a result, one of the enantiomers of compound (III) is selectively or preferentially hydrolyzed to give an optically active carboxylic acid compound [compound (IIIc)], and the other optically active form does not react and can be recovered as an optically active ester compound [compound (IIIb)].

Which optically active form is to be selectively or preferentially hydrolyzed depends on the kind of enzyme.

The asymmetric hydrolysis step is performed, for example, by mixing compound (III) and an enzyme in a solvent. The order of addition of the reagents is not particularly limited.

The enzyme to be used for the asymmetric hydrolysis step is not particularly limited as long as it can asymmetrically hydrolyze compound (III), and any enzyme derived from microorganism, animal or plant can be adopted.

The form of the enzyme for use is not particularly limited as long as the asymmetric hydrolysis ability can be maintained, and purified enzyme, crude enzyme, microorganism culture medium containing enzyme, microorganism culture containing enzyme, cell containing enzyme, or processed form thereof (e.g., freeze-dried product, acetone dry cell, milled cell, ultrasonicated cell, cell autolysate, extract, alkali-treated product etc.), or a form where enzyme is immobilized on an organic or inorganic polymer by a method known *per se* (e.g., adsorption method, polyacrylamide method, sulfur-containing polysaccharide gel method etc.) can be employed.

As such enzyme, pig liver esterase, proteases (e.g., protease derived from the genus *Aspergilus,* protease derived from the genus *Bacillus, Subtilisin carlsberg* etc.), lipases (e.g., *Candida rugosa-*derived lipase, lipase derived from the genus *Aspergilus, Candida antarctica* "A" lipase, *Candida antarctica* "B" lipase, ChiroCLEC (solid), ChiroCLEC (slurry) etc.) and the like can be mentioned, which may be used in a mixture of two or more kinds thereof.

Of these, protease derived from *Bacillus subtilis* or *Aspergillus melleus* used for food is preferable, since it is superior in steric selectivity and easily available.

These enzymes can be appropriately selected from the corresponding commercial products before use.

The amount of the enzyme to be used may be an amount exhibiting the object effect (effective amount) and the effective amount is easily determined by those of ordinary skill in the art through a simple preliminary test. For example, in the case of a commercially available protease for food, the enzyme amount is generally 0.01 g - 1 g, preferably 0.02 g - 0.1 g, per 1 g of compound (III) to be the substrate.

As the solvent, water or a mixture of water and an organic solvent within the range where the enzyme activity is not impaired can be used. The use of water alone is preferable from the industrial viewpoint. An embodiment wherein R³ is an alkyl group, particularly a methyl group, is preferable since compound (III) has high water-solubility and the step can be performed with water alone.

When an organic solvent is concurrently used, one or more kinds of N,N-dimethylformamide, dimethyl sulfoxide, THF, acetone, isopropyl acetate, ethyl acetate, methylisobutylketone (MIBK), methyl-tert-butylether (MTBE) and the like can be used.

The amount of the solvent to be used is generally 5-fold weight to 200-fold weight, preferably 10-fold weight to 100-fold weight, of compound (III).

The solvent is preferably adjusted to a pH (e.g., about pH 7.0 - 7.2) optimal to the enzyme with a buffering component such as potassium dihydrogen phosphate, potassium hydrogen phosphate, sodium acetate, sodium dihydrogen phosphate, sodium hydrogen phosphate, phosphoric acid, sodium hydroxide, potassium hydroxide and the like.

While the concentration of the buffering component in the solvent is not particularly limited as long as it does not inhibit the enzyme reaction, it is about 0.01M - 10M.

It is possible to adjust pH during reaction by appropriate addition of a base.

While the reaction temperature is not particularly limited as long as the enzyme reaction can proceed, the range optimal to the enzyme used is preferable, because too high a temperature deactivates the enzyme and too low a temperature delays the reaction rate.

For example, in the case of a protease derived from the genus *Aspergilus* or the genus *Batillus* used for foods, the reaction is carried out within the range of generally 25-40°C (preferably 28-32°C). The reaction is carried out within the above-mentioned temperature range for generally 4 hr-48 hr (preferably 8 hr-24 hr).

A mixture of compound (IIIb) and compound (IIIc) optically resolved by the asymmetric hydrolysis can be separated and purified by a conventional method (e.g., extraction, washing with water, concentration, recrystallization etc.). A method for separating these compounds utilizing the difference in the water-solubility of the compounds by partitioning between water and an organic solvent is preferable because industrial practice is easy.

To be specific, since hydrolyzed compound (IIIc) has higher water-solubility as compared to compound (IIIb) free of hydrolysis, compound (IIIb) is transferred to the organic layer by partitioning between an organic solvent selected as appropriate and water, whereby compound (IIIc) can be transferred to the aqueous layer.

In this case, since the enzyme used is also transferred to the aqueous layer, further separation and purification is necessary. In contrast, since compound (IIIb) alone is efficiently transferred to the organic layer, the compound can be used without further purification.

As the enzyme, therefore, one that does not hydrolyze the desired enantiomer but selectively or preferentially hydrolyzes unnecessary enantiomer is preferably selected.

Concretely, after the completion of the reaction, an organic solvent is evaporated as necessary, water and/or an organic solvent are/is added, the mixture is stirred, and the obtained mixture is partitioned.

The organic solvent to be used needs only to be selected appropriately in consideration of the water-solubility and the like of the compound and, for example, ethyl acetate, isopropyl acetate, toluene, MTBE, MIBK and the like and a mixed solvent thereof can be mentioned. Of these, ethyl acetate and isopropyl acetate are preferable.

The ratio of the organic solvent and water during use can be appropriately determined in consideration of the water-solubility of the obtained compound and is generally within the range of organic solvent:water (volume ratio)=1:5-5:1.

By concentrating the organic layer after washing with water as necessary, high purity compound (IIIb) can be obtained. On the other hand, compound (IIIc) can be recovered from the aqueous layer by, after concentration, washing the residue with ethanol and collecting the residue by filtration.

Of the asymmetric hydrolysis steps, an embodiment where R³ of compound (III) is an alkyl group is novel and industrially more preferable as compared to an embodiment where R³ is an aryl group, since (1) thioamides (e.g., thioacetamide) as starting materials are obtained at a low cost; (2) the substrate has high water-solubility, and co-use of an organic solvent for the enzyme reaction is not necessary; and (3) acetic acid and the like resulting from a side reaction due to the deprotection with an acid can be easily removed from the object product; and other reasons.

### 3. Alkali hydrolysis step

Compound (IIId) and compound (IIIf), which are optically active carboxylic acid compounds, can be obtained by hydrolyzing, with an alkali, an ester moiety of compound (IIIb) optically resolved by asymmetric hydrolysis, and optically active compound (IIIe) obtained using optically active compound (I) in a thiazoline cyclization step.

Since the optically active carboxylic acid compounds are crystals, they are characterized in that they can be easily purified by slurry washing with a solvent, recrystallization, formation of salt with amine and the like. Depending on the conditions, moreover, the optical purity can be improved.

Specifically, optically active ester compound (III) and alkali are mixed in a solvent. The order of addition of the reagents is not particularly limited.

As the alkali to be used, aqueous sodium hydroxide solution, aqueous potassium hydroxide solution, aqueous cesium hydroxide solution, aqueous lithium hydroxide solution and the like can be mentioned. An aqueous sodium hydroxide solution is preferable, and 6-8M aqueous sodium hydroxide solution is preferably used.

The amount of alkali to be used is generally 1 - 1.2 mol, preferably 1.02 - 1.05 mol, per 1 mol of optically active ester compound (III).

As the solvent, a hydrophilic solvent is preferable, and methanol, ethanol, 1-propanol, 2-propanol, tert-butanol, tetrahydrofuran (THF), DMSO, DMF, DMA and the like are preferable. They may be used in a mixed solvent of two or more kinds thereof.

The amount of the solvent to be used is 1- to 10-fold weight, preferably 2- to 5-fold weight, relative to optically active ester compound (III).

The alkali hydrolysis step is performed at a temperature within the range of generally from 20°C to the refluxing temperature of the solvent to be used (preferably 25°C-30°C). The reaction time is generally 0.5 hr-2 hr (preferably 0.5 hr-1 hr) within the above-mentioned temperature range.

After completion of the reaction, compound (IIId) or compound (IIIf) contained in the reaction mixture can be isolated and purified by an isolation and purification method of carboxylic acid derivative, which is generally employed by those of ordinary skill in the art.

For example, after completion of the reaction and adjusting the pH to 6.5-7.5 with an acid such as acetate acid, hydrochloric acid, sulfuric acid and the like, the compound can be purified by (1) extraction with an organic solvent, washing with water, and concentration for isolation, followed by crystallization and the like; or (2) concentration of the mixture, followed by slurry washing with a solvent, recrystallization, formation of salt with amine and the like.

Of the optically active compounds (III) obtained by the thiazolidine cyclization step, asymmetric hydrolysis step or alkali hydrolysis step, an embodiment wherein R¹ is a hydrogen atom or a methyl group, R² is a methyl group and R³ is an alkyl group, an allyl group or an amino group, namely, compound (IIII) ; and an embodiment wherein R¹ is a hydrogen atom, R² is an alkyl group and R³ is an alkyl group, an allyl group or an amino group, namely, compound (IIIm) are novel compounds and useful as synthetic intermediates for pharmaceutical products.

### 4. Deprotection step

Optically active α-alkylcysteine compound [compound (IV)] can be obtained by treating optically active thiazolinecarboxylic acid compound (IIId) or compound (IIIf) with an acid to deprotect a thiazolidine ring.

Specifically, optically active compound (IIId) or compound (IIIf) and an acid are mixed in a solvent. The order of addition of the reagents is not particularly limited.

As the acid to be used, hydrochloric acid, sulfuric acid, acetic acid, trifluoroacetic acid and the like can be mentioned, with preference given to hydrochloric acid. Use of concentrated hydrochloric acid is more preferable. The amount of the acid to be used is generally 0.1 - 50 mol, preferably 10 - 30 mol, per 1 mol of optically active thiazolinecarboxylic acid compound (III).

As the solvent, water, alcohols (e.g., methanol, ethanol etc.) and a mixed solvent thereof can be mentioned, with preference given to water. When hydrochloric acid is used as the acid, water contained in the acid is included in the solvent.

The amount of the solvent to be used is generally 0.5-fold weight to 20-fold weight, preferably 5-fold weight to 10-fold weight, of optically active thiazolinecarboxylic acid compound (III).

The reaction temperature is within the range of generally from 30°C to the refluxing temperature of the solvent to be used (preferably refluxing). The reaction is carried out generally for 0.5 hr-48 hr (preferably 1 hr-24 hr) within the above-mentioned temperature range.

After completion of the reaction, optically active compound (IV) contained in the reaction mixture can be isolated and purified by an isolation and purification method known per se of amino acid derivative, which is generally employed by those of ordinary skill in the art for amino acid synthesis. For example, optically active compound (IV) is preferably isolated and purified in the form of a salt with an acid used for deprotection with an acid.

Specifically, an acid addition salt of compound (IV) can be isolated by (1) concentrating and drying the reaction mixture, and filtering or washing the residue with a suitable organic solvent (e.g., toluene, isopropyl acetate etc.); or (2) partially concentrating the reaction mixture, and filtering or washing the crystal precipitated after cooling; and the like.

In addition, compound (IIIb), compound (IIIe) and compound (IIIh) can be lead to compound (IV) by direct reaction with an acid without alkali hydrolysis. In this case, compound (IIIb), compound (IIIe) or compound (IIIh) is used as a starting material instead of compound (IIId) or compound (IIIf) for the treatment under the same conditions as in the above-mentioned deprotection.

The thus-obtained compound (IV) is a useful synthetic intermediate for pharmaceutical products. For example, α-methyl-L-cysteine hydrochloride can be lead to S-[2-(ethanimidoylamino)ethyl]-2-methyl-L-cysteine, which is an anti-inflammatory agent having a nitrogen oxide synthase inhibitory activity, by the method described in WO2004/039772.

### EXAMPLES

The present invention is explained in detail in the following by way of Examples, which are not to be construed as limitative.

### Example 1: Synthesis of 2-amino-4-methylthiazoline-4-carboxylic acid

An aqueous solution (30 mL) of α-chloromethylenealanine hydrochloride (10.0 g, 58 mmol) was adjusted to pH=7 with aqueous sodium hydroxide solution, thiourea (5.0 g, 65 mmol) was added, and the mixture was stirred at 100°C for 5 hr. Under reduced pressure, water was evaporated, ethanol was added, and the solvent was evaporated under reduced pressure. Ethanol (100 mL) was added, and the mixture was stirred at 50°C. The reaction mixture was filtered and the filtrate was concentrated and dried to give the object title compound (11.29 g).
¹H-NMR(400MHz, CD₃OD) δ 1.62(s, 3H), 3.54 (dd, 2H, J=7.1 Hz, 61.2Hz);
¹³C-NMR(100MHz, CD₃OD) δ 180.57, 174.92, 74.78, 43.21, 25.59; MS(FAB) m/z 161[M⁺+H].

### Example 2: Synthesis of methyl 4-methyl-2-phenylthiazoline-4-carboxylate

To a solution of α-chloromethylenealanine methyl ester (84 mg, 0.55 mmol) in methanol (1 mL) was added thiobenzamide (69 g, 0.50 mmol), and the mixture was stirred at 60°C for 16 hr. Production of the object title compound (94 mg) was confirmed by quantitation by HPLC.

### Example 3: Synthesis of methyl 2,4-dimethylthiazoline-4-carboxylate

To a solution of α-chloromethylenealanine methyl ester (7.0 g, 46.18 mmol) in methanol (35 mL) was add thioacetamide (2.79 g, 37 mmol), and the mixture was stirred at 60°C for 16 hr. Under reduced pressure, methanol was evaporated, and ethyl acetate (70 mL) and water (35 mL) were added. The mixture was partitioned and the organic layer was washed with brine (20 mL), concentrated and vacuum dried to give the object title compound (6.26 g).
¹H-NMR(400MHz, CD₃OD) δ 1.50(s, 3H), 2.22(s, 3H), 3.54(dd, 2H, J=7.2Hz, 126.1Hz), 3.76(s, 3H);
¹³C-NMR(100MHz, CD₃OD) δ 175.10, 172.17, 85.33, 54.09, 43.56, 24.54, 20.33;
MS (FAB) m/z 161 [M⁺+H] .

### Example 4: Synthesis of methyl (R)-2,4-dimethylthiazoline-4-carboxylate

To a solution of methyl 2,4-dimethylthiazoline-4-carboxylate (1.0 g, 5.78 mmol) in aqueous potassium phosphate (pH=7.2, 100 mL) was added food protease (10%, 0.5 g, manufactured by Amano, derived from the genus *Bacillus*), and the mixture was stirred at 30°C for 8 hr. Then, ethyl acetate (50 mL) was added, and the mixture was partitioned. The organic layer was washed with brine (5 mL), concentrated and vacuum dried to give the object title compound (0.40 g, optical purity 98%ee).
HPLC conditions
column: DAICEL CHIRALCEL OD-H;
column temperature: r.t.
mobile phase: haxane:EtOH=95:5
detection wavelength: 210 nm;
flow rate: 1.0 mL/min.

### Example 5: Synthesis of (R)-2,4-dimethylthiazoline-4-carboxylic acid

To a solution of methyl (R)-2,4-dimethylthiazoline-4-carboxylate (1.95 g, 11.26 mmol) in ethanol (9.0 mL) was added 6M aqueous sodium hydroxide solution (1.98 mL, 11.88 mol), and the mixture was stirred at 30°C for 1 hr. After completion of the reaction, the mixture was adjusted to pH=7.0 with acetic acid and concentrated. Ethanol (11.5 mL) was added and the mixture was concentrated. This operation was repeated twice and ethanol (23 mL) was added. The mixture was slurry washed and filtered. The crystals were dried to give the object title compound (1.42 g).
¹H-NMR (400MHz, D₂O) δ 1.31(s, 3H), 2.22(s, 3H), 3.40(dd, 2H, J=7.1 Hz, 92.4Hz);
¹³C-NMR(100 MHz, D₂O) δ 181.63, 170.03, 85.39, 54.09, 43.78, 24.25, 19.66;
MS(FAB) m/z 147 [M⁺+H] .

### Example 6 Synthesis of (R)-methylcysteine hydrochloride

A solution of (R)-2,4-dimethylthiazoline-4-carboxylic acid (1.35 g, 8.48 mmol) in concentrated hydrochloric acid (13.5 mL) was stirred at 100°C for 20 hrs. Thereafter, the mixture was concentrated under reduced pressure, the obtained solid was washed with toluene, collected by filtration and dried to give the object title compound (0.28 g). The spectrum data matched with those in known references. The object product was benzyloxycarbonylated and subjected to HPLC analysis to find an optical purity of not less than 99% ee.
HPLC conditions
column: DAICEL CHIRALCEL OD-RH;
column temperature: r.t.
mobile phase: 0.03 M KH₂PO₄(pH=2):MeCN=60:40
detection wavelength: 210 nm;
flow rate: 1.0 mL/min.

## Claims

1. A production method of a compound represented by the formula (III) : 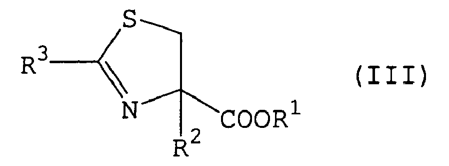 wherein R¹ is a hydrogen atom or an alkyl group, R² is an alkyl group, and R³ is an alkyl group, an allyl group, an aryl group or an amino group, or a salt thereof, which comprises reacting a compound represented by the formula (I): 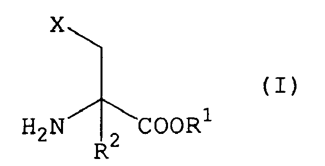 wherein R¹ and R² are as defined above, and X is a chlorine atom, a bromine atom or an iodine atom, or a salt thereof with a compound represented by the formula (II): 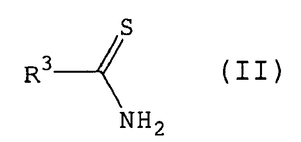 wherein R³ is as defined above.

2. The production method of claim 1 wherein the compound represented by the formula (I) is a racemate.

3. The production method of claim 2 wherein R¹ is an alkyl group.

4. A production method of a compound represented by the formula (IIIb): 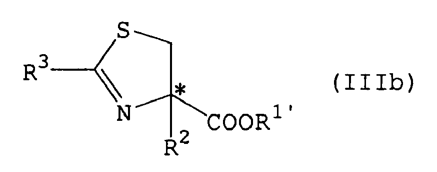 wherein R¹' and R² are the same or different and each is an alkyl group, R³ is an alkyl group, an allyl group, an aryl group or an amino group, and * shows a chiral carbon atom, or a salt thereof and a compound represented by the formula (IIIc): 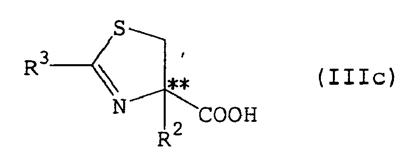 wherein ** shows a chiral carbon atom of an S configuration when * shows an R configuration, and a chiral carbon atom of an R configuration when * shows an S configuration, and other symbols are as defined above, or a salt thereof, which comprises reacting a compound represented by the formula (IIIa) : 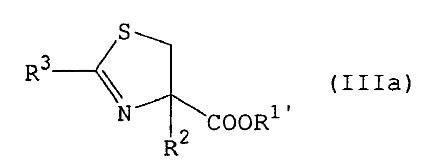 wherein each symbol is as defined above, which is obtained by the method described in claim 3, or a salt thereof with an enzyme having an ability to asymmetrically hydrolyze the compound.

5. A production method of a compound represented by the formula (IIId) : 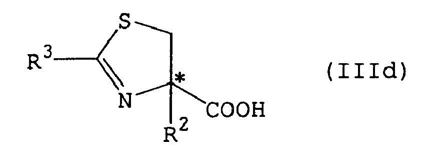 wherein each symbol is as defined in claim 4, or a salt thereof, which comprises alkali hydrolysis of an ester moiety of a compound represented by the formula (IIIb): 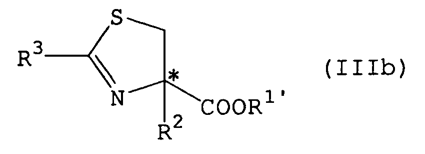 wherein each symbol is as defined in claim 4, which is obtained by the method described in claim 4, or a salt thereof.

6. A production method of a compound represented by the formula (IVa) : 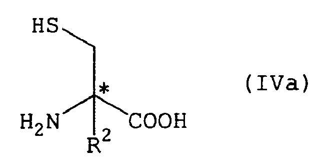 wherein R² is as defined in claim 5, or a salt thereof, which comprises reacting a compound represented by the formula (IIId) : 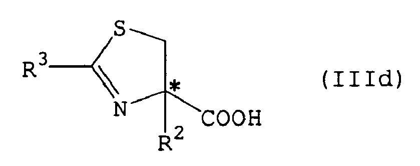 wherein each symbol is as defined in claim 5, which is obtained by the method described in claim 5, or a salt thereof with an acid for deprotection.

7. A production method of a compound represented by the formula (IVb) : 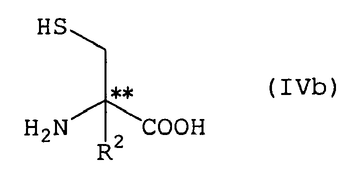 wherein R² is as defined in claim 4, or a salt thereof, which comprises reacting a compound represented by the formula (IIIc): 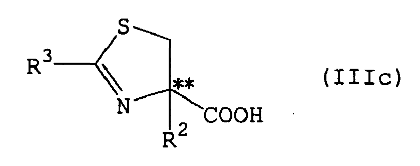 wherein each symbol is as defined in claim 4, which is obtained by the method described in claim 4, or a salt thereof with an acid for deprotection.

8. The production method of any one of claims 1 to 7, wherein R³ is a methyl group.

9. The production method of any one of claims 4 to 8, wherein R^{1'} is a methyl group or an ethyl group.

10. The production method of any one of claims 1 to 9, wherein R² is a methyl group.

11. The production method of claim 1, wherein the compound represented by the formula (I) is an optically active form.

12. The production method of claim 11, wherein R¹ is a hydrogen atom.

13. The production method of claim 11, wherein R¹ is an alkyl group.

14. A production method of a compound represented by the formula (IIIf): 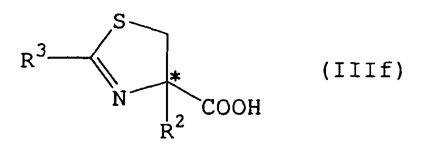 wherein R² is an alkyl group, R³ is an alkyl group, an allyl group, an aryl group or an amino group, and * shows a chiral carbon atom, or a salt thereof, which comprises alkali hydrolysis of an ester moiety of a compound represented by the formula (IIIe): 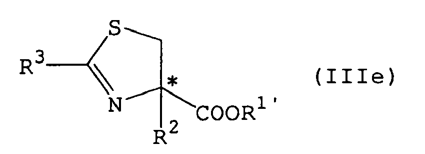 wherein R^{1'} is an alkyl group, and other symbols are as defined above, which is obtained by the method described in claim 13, or a salt thereof.

15. A production method of a compound represented by the formula (IV): 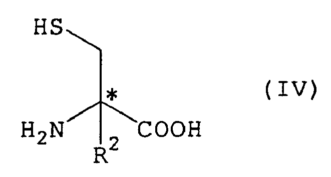 wherein R² is as defined in claim 14, or a salt thereof, which comprises reacting a compound represented by the formula (IIIf) : 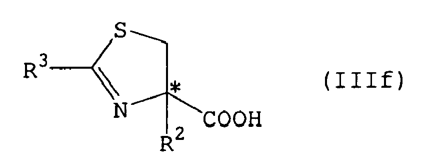 wherein each symbol is as defined in claim 14, which is obtained by the method described in claim 12 or 14, or a salt thereof, with an acid for deprotection.

16. The production method of any one of claims 11 to 15, wherein R³ is a methyl group.

17. A production method of a compound represented by the formula (IIIh): 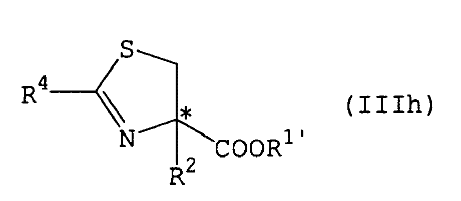 wherein R¹', R² and R⁴ are the same or different and each is an alkyl group, and * shows a chiral carbon atom, or a salt thereof and a compound represented by the formula (IIIi): 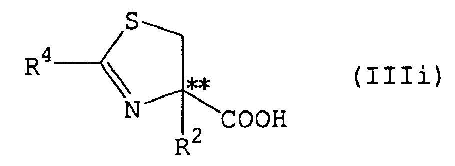 wherein ** shows a chiral carbon atom of an S configuration when * shows an R configuration, and a chiral carbon atom of an R configuration when * shows an S configuration, and other symbols are as defined above, or a salt thereof, which comprises reacting a compound represented by the formula (IIIg) : 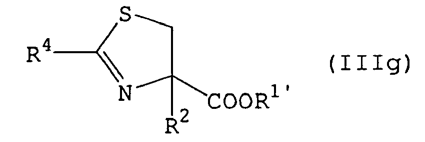 wherein each symbol is as defined above, with an enzyme having an ability to asymmetrically hydrolyzing the compound.

18. A production method of a compound represented by the formula (IIIj): 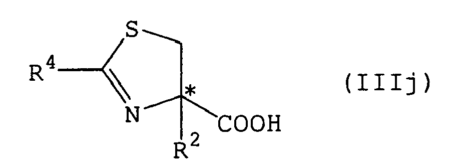 wherein each symbol is as defined in claim 17, or a salt thereof, which comprises alkali hydrolysis of an ester moiety of a compound represented by the formula (IIIh): 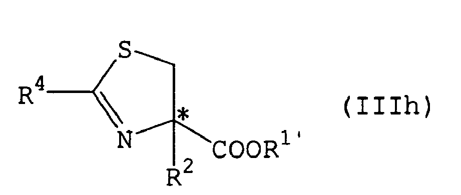 wherein each symbol is as defined in claim 17, which is obtained by the method described in claim 17, or a salt thereof.

19. A production method of a compound represented by the formula (IVa) : 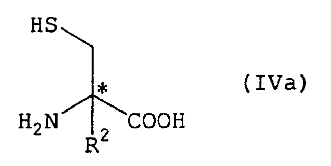 wherein R² is as defined in claim 18, or a salt thereof, which comprises reacting a compound represented by the formula (IIIj): 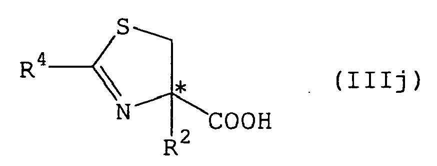 wherein each symbol is as defined in claim 18, which is obtained by the method described in claim 18, or a salt thereof with an acid for deprotection.

20. A production method of a compound represented by the formula (IVb): 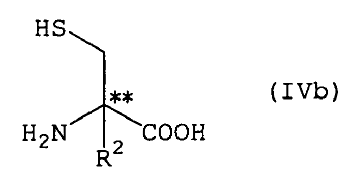 wherein R² is as defined in claim 17, or a salt thereof, which comprises reacting a compound represented by the formula (IIIi) : 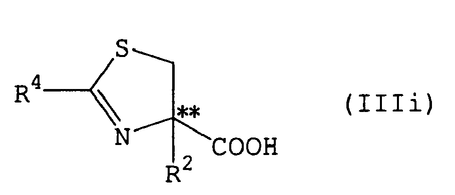 wherein each symbol is as defined in claim 17, which is obtained by the method described in claim 17, or a salt thereof with an acid for deprotection.

21. The production method of any one of claims 17 to 20, wherein R³ is a methyl group.

22. The production method of any one of claims 17 to 21, wherein R¹' is a methyl group or an ethyl group.

23. The production method of any one of claims 17 to 22, wherein R² is a methyl group.

24. A compound represented by the formula (IIIk): 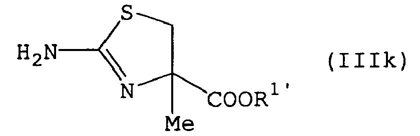 wherein R¹' is an alkyl group, or a salt thereof.

25. A compound represented by the formula (IIIl): 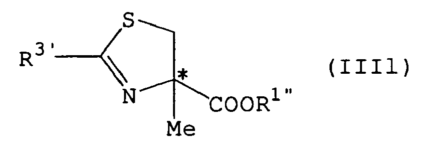 wherein R¹" is a hydrogen atom or a methyl group, R³, is an alkyl group, an allyl group or an amino group, and * shows a chiral carbon atom, or a salt thereof.

26. A compound represented by the formula (IIIm): 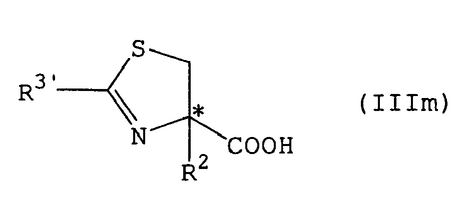 wherein R² is an alkyl group, R³' is an alkyl group, an allyl group or an amino group, and * shows a chiral carbon atom, or a salt thereof.
